# EUROPÄISCHE PATENTANMELDUNG

(11) **EP 3 978 000 A1**
(43) Veröffentlichungstag der Anmeldung: **06.04.2022**
(21) Anmeldenummer: 21401037.3
(22) Anmeldetag: 30.09.2021
(51) Int. Cl.: A61K 31/575, A61P 31/14

(54) **DESOXYCHOLSÄURE SOWIE DEREN VERBINDUNGEN ZUR VERWENDUNG BEI DER BEHANDLUNG VON ERKRANKUNGEN**

(30) Priorität: 02.10.2020 DE 102020006049
(71) Anmelder: Vlcek, Radim, 83209 Prien am Chiemsee (DE)
(72) Erfinder: Vlcek, Radim, 83209 Prien am Chiemsee (DE)

(57) **Zusammenfassung**

Gegenstand der Erfindung ist der Einsatz von Desoxycholsäure sowie deren Verbindungen zur Verwendung bei der Behandlung von Erkrankungen mit Corona-Viren (CoV).

Coronaviren lösen grippeähnliche Atemwegserkrankungen aus. Speziell das SARS-CoV-2 hat sich zu einer weltweiten Pandemie ausgebreitet. Bis zum heutigen Zeitpunkt besteht keine etablierte therapeutische Behandlungsmöglichkeit einer Virämie mit einem Coronavirus - man ist auf Impfungen und Hygienemaßnahmen angewiesen.

Die Aufgabe der Erfindung besteht in der Hebung der Wirkkraft des unspezifischen Immunsystems zu Beginn einer CoV-Erkrankung (etwa Tag 1.-4.). Dadurch soll auf einen milderen sowie verkürzten Krankheitsverlauf hingewirkt werden.

Als Wirkstoff einer Formulierung zur Behandlung einer CoV-Erkrankung wird die freie Desoxycholsäure (C₂₄H₄₀O₄, CAS: 83-44-3) in pharmazeutisch wirksamer Menge eingesetzt, oder deren zielführende Verbindungen. Desoxycholsäure besitzt die Fähigkeit zur Stimulierung des unspezifischen Immunsystems, darunter auch der Makrophagen.

Die erfindungsgemäße Anwendung von Desoxycholsäure in der ersten Phase einer CoV-Erkrankung erschließt die Möglichkeit, die aufkeimende Virämie vor der Phase der pulmonalen Manifestation zu behandeln. Gelingt hier eine effektive Intervention, wird der Krankheitsverlauf gemildert und die Anzahl der schweren Erkrankungsfälle gesenkt. Infolgedessen wird die Liegezeit der Erkrankten verkürzt, was für den Patienten, die Gesundheitssysteme sowie Volkswirtschaften entscheidend positive Folgen hätte.

## Beschreibung

### Anwendungsgebiet

Gegenstand der Erfindung ist der Einsatz von Desoxycholsäure sowie deren Verbindungen (pharmazeutisch geeignete Salze und Konjugate) zur Verwendung bei der Behandlung von Erkrankungen mit Corona-Viren (CoV). Die Anwendungsgebiete beziehen sich auf die Humanmedizin sowie Tiermedizin.

### Stand der Technik

Coronaviren (CoV) sind hinsichtlich deren Klassifikation eine Gruppe von umhüllten Viren mit einer einzelsträngigen Plusstrang-RNA, welche menschliche und tierische Zellen als Wirtszellen befallen können (1). Während Influenzaviren die klassische Grippe verursachen, lösen Coronaviren grippeähnliche Atemwegserkrankungen aus.

Bis ca. 2002 sind Coronaviren typischerweise mehr mit Erkältungssymptomen aufgetreten. Seitdem aber wurden schwere Atemwegsbeschwerden registriert, denen die Namensgebung SARS (severe acute respiratory syndrome) geschuldet ist. Eine größere Welle gab es auch etwa 2012 in Saudi-Arabien mit dem MERS-CoV (2).

Im Dezember 2019 entstand von Wuhan (China) aus erneut eine epidemiologische Welle, mit dem neuartigen Coronavirus SARS-CoV-2, welches eine ähnliche Infektiosität wie die oben benannten Corona-Viren (3). Es ist ein Beta-Coronavirus, seine genetische Sequenz ist zu 97,5 % mit dem SARS-CoV identisch (4). Der Ursprung des Virus wird auf eine Fledermausart zurückgeführt (5).

Die Übertragung von Coronaviren erfolgte in der Vergangenheit vermutlich von Tier auf den Menschen. Es wird aktuell erforscht, wie weit eine Übertragung zwischen Tier und Mensch in beide Richtungen erfolgt sowie welche Tierarten sich anstecken, oder auch selbst erkranken können (6).

Der Hauptübertragungsweg von SARS-CoV-2 ist die respiratorische Aufnahme von Flüssigkeitspartikeln, welche das Virus beinhalten. Dies ist etwa beim Husten und Niesen der Fall, aber auch beim Singen oder Schreien. Auch eine Kontaktübertragung ist nicht auszuschließen (7). Der Manifestationsindex von COVID-19 liegt je nach Studie bei 55-85% (7). Hinsichtlich der Symptomatik entwickelt eine SARS-CoV-2 Infektion typische Symptome. In chinesischen Studien wurden zu Beginn häufig Fieber, Husten, eine allgemeine Erschöpfung (Krankheitsgefühl) und teils auch Durchfall beobachtet - im Verlauf der Erkrankung können Funktionsstörungen (und Schädigungen) verschiedener Organe hinzukommen, vor allem der Lunge (8). In Deutschland wurden als Kernsymptome trockener Husten (45%), Fieber (38%), Schnupfen (20%), Geruchs- und Geschmacksstörungen (15%) beobachtet (7). Langzeitfolgen konnten aktuell nicht untersucht werden. Die WHO hat die vom SARS-CoV-2 verursachte Erkrankung am 11.2.2020 als COVID-19 (Corona Virus Disease 2019) benannt (9).

Studien berichten, dass etwa 81% aller Erkrankten einen milden Verlauf haben, etwa 14% einen schweren und etwa 5% einen kritischen (10). Offensichtlich besteht ein großer Unterschied in der Fähigkeit einzelner Erkrankter immunologische auf eine Infektion mit dem SARS-CoV-2 zu reagieren.

Seit dessen ersten registrierten Auftreten im Dezember 2019 hat sich das SARS-CoV-2 Virus bis aktuell September 2021 weltweit zu einer Pandemie ausgebreitet. Zum 20.09.2021 bestand weltweit eine globale Fallzahl von 228.662.524 mit 4.694.251 an oder mit COVID-19 Verstorbenen (11). Die gesundheitlichen und ökonomischen Folgen dieser weltweiten Pandemie sind hinlänglich bekannt.

Desoxycholsäure (int. Abkürzung DCA von DeoxyCholicAcid) ist eine körpereigene sekundäre Gallensäure C₂₄H₄₀O₄. Sie entsteht im enterohepatischen Kreislauf aus dem Ausgangsprodukt Cholsäure durch Umbauprozesse von verschiedenen Bakterien im Darm. Der größte Anteil der körperproduzierten Desoxycholsäure findet sich im Körper gebunden als Tauro-DCA und Glyco-DCA wieder (12). Desoxycholsäure stellt somit einen körpereigenen Stoff mit eigenem Kreislauf und Regulationsmechanismen dar.

Die älteste bekannte Funktion der Gallensäure Desoxycholsäure ist ihre Funktion in der Fettverdauung. Darauf basieren mehrere Verwendungsansätze für Desoxycholsäure in der Nahrungsmittelindustrie und Pharmazie:
- Bis in die siebziger Jahre hinein wurde Desoxycholsäure in Nahrungsmitteln als Emulgator verwendet. Formell besteht seitens der WHO eine Unbedenklichkeit mit einem ADI (acceptable daily intake) von bis zu 1,25 mg/kg Körpergewicht (13).
- Seit längerem besteht eine Verwendung von DCA in Choleretika mit dem Ziel, den Gallenkreislauf zu stabilisieren, oder die Fettverdauung zu verbessern (12). Desoxycholsäure als Choleretikum ist auf dem aktuellen Pharma-Markt nicht aktuell, jedoch als diesbezüglicher Wirkstoff nicht verschwunden (Pat. ZA 200407911B, 2002).
- In den letzten Jahren wurde Desoxycholsäure als Wirkstoff zur Behandlung von Fettansammlungen unter der Haut in der kosmetischen Medizin ("Fett-Weg-Spritze") patentiert (Pat. US 20170319601, 2017) und (Pat US 20170119794, 2017) sowie zugelassen (14). In diesem Bereich stoßen auch andere Patente zum Fettabbau dazu wie (Pat. US 2006222695A1, 2005) und (Pat. CN 110302082A, 2018).

Als weitere Einsatzmöglichkeiten für Desoxycholsäure in der Medizin wurden folgende Felder publiziert oder patentiert:
- Verwendung von DCA zusammen mit anderen Gallensäuren gegen Darmpilze, vor allem Candida (Pat. US 4681876A, 1984).
- Modulation von Entzündungsprozessen: Je nach Szenario agiert DCA als entzündungssenkendes Agens (15) (16), als Entzündungspromotor (17), oder direkt als Agens gegen Entzündungen (Pat. KR 101313894B1, 2011) sowie bei Wundheilungsstörungen (Pat. DE 3546360A1, 1985).
- Einsatz von DCA zur Bekämpfung von Akne sowie Stimulierung der Hautregeneration mit Kollagenbildung (Pat. KR 102059854B1, 2013).
- Wirkung von DCA auf Reparaturprozesse bei Haarfollikeln (Pat. WO 2018030560A1, 2016).
- Verwendung von DCA als Appetitzügler für Adipositas-Patienten (Pat. DE 102017119313A1, 2017).
- Verwendung von DCA gegen Muskelschwäche (Pat. KR 20200022789A, 2018).

Die für diese Patentanmeldung wesentliche Funktion von Desoxycholsäure ist deren Wirkung als Immunmodulator. Diese wird seit den 70er Jahren erforscht. Desoxycholsäure besitzt die Fähigkeit zur Stimulierung des unspezifischen Immunsystems (18), darunter auch der Makrophagen (19), (20). In den letzten 10 Jahren wurde die Forschung hierzu verstärkt und der immunologisch steuernde Einfluss einzelner Gallensäuren konnte etwas näher beschrieben werden auf:
- Rezeptoren im Immunsystem (21),
- Immunmodulation bei Entzündungen (22), (23) sowie
- die konkrete Stimulation des unspezifischen Immunsystems und Makrophagenaktivierung (24).

Eine immunmodulierende Fähigkeit im Sinne einer antiviralen Wirkung konnte bei Desoxycholsäure für bestimmte Indikationen aufgezeigt werden. So konnte dies bei Herpes Labilalis (25) und Herpes Zoster (26) klinisch nachgewiesen werden. Diese Wirkung von DCA ist jedoch nur für manche Viruserkrankungen existent - bei anderen besteht keine Wirksamkeit.

Für die Verwendung von Desoxycholsäure als Immunmodulator bestehen einige Publikationen und klinische Studien, jedoch aktuell keine Zulassung als Medikament.

Überdies stehen die Gallensäuren Desoxycholsäure (DCA) und Ursodesoxycholsäure (UDCA) in einem antagonistischen immunologischen Wechselspiel, etwa innerhalb der Wundheilung (27). UDCA wird ein antiphlogistischer und immunsuppressiver Nutzen zugeschrieben (28), bei DCA wird im Gegenteil eine Funktion in der Entzündungsstimulation vermutet.

In der traditionellen chinesischen Medizin wird "Bärengalle" im Einsatz gegen COVID-19 versucht (29). Diese enthält einen (variierend) hohen Anteil an UDCA (30), womit deren immunsuppressive Wirkung auch bei COVID-19 in den Blickwinkel fällt. Immunsuppression ist zu Beginn einer COVID-19 Erkrankung kontraindiziert und gibt erst in einer etwaigen (seltenen) pneumonalen Phase sowie bei schweren Verläufen (etwa "cytokine storm") einen Sinn, was auch in Forscherkreisen so gesehen wird (31) (32).

Diese Patentanmeldung verfolgt jedoch einen völlig neuen sowie doppelt anderen Weg. Hier wird die immunstimulierende Eigenschaft der Desoxycholsäure in der initialen Phase einer COVID-19 Erkrankung genutzt - statt (wie Stand der Forschung) die Idee der Immunsuppression mit UDCA in der finalen Phase von COVID-19. Diese Wirkung ist nur mit einem isolierten Präparat auf reiner Desoxycholsäure-Basis erreichbar. Natürlich oder naturidentisch zusammengesetzte Gallenextrakte - auch die "Bärengalle" - können so nicht wirken, da sich die Komponenten DCA und UDCA gegenseitig sowie im Wechselspiel mit weiteren enthaltenen Gallensäuren in der Wirkung aufheben. Daher wurde auch beim Einsatz von natürlichen Gallenextrakten in der Forschergemeinde keine relevante Wirkung auf COVID-19 registriert und der Ansatz mit Gallenextrakten (leider) nicht weiterverfolgt.

Bis zum heutigen Zeitpunkt besteht keine länderübergreifend zugelassene therapeutische Behandlungsmöglichkeit der Grunderkrankung (Virämie) mit einem Coronavirus (33), COVID-19 eingeschlossen.

Hinsichtlich COVID-19 hat in der EU bisher einzig der Wirkstoff Remdesivir eine bedingte Zulassung zur Behandlung erhalten (34). Die Indikation besteht für an COVID-19 erkrankte Patienten, welche bereits eine Pneumonie (im Anfangsstadium) entwickelt haben und Sauerstoffzufuhr brauchen (35). Die Zulässigkeit einer Therapie mit Remdesivir ist somit erst in der zweiten Phase der Erkrankung zulässig. Wirksamkeitsnachweise gibt es für die Verkürzung der Genesungsdauer, jedoch keine statistische Signifikanz für einen Überlebensvorteil (36). Insgesamt ist somit die Behandlung mit Remdesivir bislang keine hinreichende Lösung der Behandlungsfrage für COVID-19 Patienten.

Weitere Ansätze in Erprobung zur COVID-19 Therapie sind in der Übersicht (37) und (35):
- Erprobung von antiviralen Mitteln im Off-Label-Use (neben dem obigen Remdesivir) wie Pinavir / Ritonavir / Lopinavir, Ribavirin, Camostat, Favipiravir (T-705), Oseltamivir, Chloroquine, Interferon),
- Gabe von Rekonvaleszenten-Plasma,
- Behandlung von begleitenden bakteriellen Superinfektionen und/oder septischem Verlauf wie Amoxicillin, Azithromycin, Fluoroquinolone,
- Symptombehandlung (wie Sauerstofftherapie, fiebersenkende Mittel, Schmerzmittel, etc.).
- Neueste Forschungen zum aktuellen SARS-CoV-2 erforschen eine Immunschwäch in der "Vorhut der Virusabwehr". Gesucht wird im Funktionsbereich der Botenstoffe, etwa in der Interferonhemmung (38).

Keiner der obigen Ansätze brachte den notwendigen Behandlungserfolg, die meisten Ansätze sind im Hypothesenstadium. Aufgrund einer mangelnden Behandlungsmöglichkeit wird weltweit der Fokus auf zwei andere Interventionen gesetzt: Impfungen sowie Hygienemaßnahmen. Beide Ansätze sind mit immensen Kosten verbunden.

Aktuell sind in Deutschland vier Impfstoffe gegen SARS-CoV-2 zugelassen (39), weltweit gibt es weitere mit länderspezifischer Zulassung sowie Kandidaten (40). So wesentlich wie eine Impfung in der Pandemiebekämpfung ist, hat diese Grenzen. Der Impfschutz schwindet rapide mit der (bereits mehrfach erfolgten) Mutation des Erregers (41). Zudem ist eine wiederholende Auffrischung der Impfung nötig - aktuell werden in der EU 6 Monate bei mRNA-Impfstoffen diskutiert. Um einen dauerhaften Schutz gegen COVID-19 zu gewährleisten und zugleich immense Kosten durch fortwährende Impfungen zur Auffrischung oder neue gegen Mutationen zu vermeiden, ist die Entwicklung eines Medikaments zur Behandlung von COVID-19 unerlässlich.

### Kurzbeschreibung der Erfindung - Aufgabe und Nutzen

Aufgrund des Fehlens einer nachweislich wirksamen Behandlungsmöglichkeit für Erkrankungen mit Coronaviren ist es offensichtlich, dass eine jedwede mögliche Behandlungsmöglichkeit einer Erkrankung mit einem Coronavirus, sei es auch nur in Teilen, von höchstem internationalem medizinischem und wirtschaftlichem Interesse wäre.

Das Ziel der Erfindung ist es, die Wirkkraft des unspezifischen Immunsystems zu steigern, welches vor allem zu Beginn einer Erkrankung mit einem Coronavirus (inklusive SARS-CoV-2) die erste Immunbarriere gegen diesen Erreger bildet. Die Aufgabe der Erfindung besteht in der Hebung der körpereigenen Immunabwehr innerhalb der initialen (infektiösen) Erkrankungsphase (1.-4. Tag), wo Virusreplikation stattfindet (42).

Die Aufgabe der Stärkung der körpereigenen unspezifischen Immunabwehr wird dadurch erreicht, dass dem Erkrankten Desoxycholsäure oder deren pharmazeutisch zielführende Verbindungen in pharmazeutisch wirksamer Menge verabreicht werden. Im Falle der Wahl von Verbindungen der Desoxycholsäure, werden deren pharmazeutisch verwertbare Salze oder zuckergebundene Konjugate verwendet.

Die obige erfindungsgemäße Anwendung von DCA erschließt die Möglichkeit, eine CoV-Virämie zum Krankheitsbeginn zu behandeln. Ein milderer und kürzerer Verlauf würde nicht nur persönliches Leid (sowie die Sterberate) senken, sondern auch eine Entlastung des Gesundheitssystems bedeuten mit allen positiven Folgen für die betreffende Volkswirtschaft.

Dieser Einsatz der Erfindung ist praktikabel in der Humanmedizin, als auch in der Veterinärmedizin bei Säugetieren mit einem Immunsystem, welches wie beim Menschen Desoxycholsäure zur Immunmodulation nutzt. Die beschriebene Erfindung eröffnet und daher eine Behandlungsmethode, welche sowohl in der Human-, wie auch in der Veterinärmedizin (v. a. für Nutztierarten) anwendbar ist.

### Kurzbeschreibung der Erfindung - Praktische Umsetzung

Als Wirkstoff einer Formulierung zur Behandlung einer CoV-Erkrankung wird primär die freie Desoxycholsäure (DCA, C₂₄H₄₀O₄, CAS: 83-44-3) eingesetzt.

Als alternativer oder komplementärer Wirkstoff in einer erfindungsgemäßen Formulierung können neben Desoxycholsäure auch deren pharmazeutisch verwertbare Salze eingesetzt werden, da Desoxycholsäure im körpereigenen Gleichgewicht teils als Salz vorliegt. Bevorzugt wird hier Natrium-Desoxycholat (NaDCA, C₂₄H₃₉NaO₄, CAS: 302-95-4), welches naturgemäß im Körper vorgefunden wird und in einer erfindungsgemäßen Formulierung eingesetzt werden kann.

Der Großteil der in unserem Körper produzierten Desoxycholsäure wird durch bakterielle Prozesse (Konjugationsbakterien) in zuckergebundenen Konjugaten gespeichert. Die relevanten sind hier Glyco-DCA (GDCA, C₂₆H₄₃NO₅, CAS: 360-65-6) und Tauro-DCA (TDCA, C₂₆H₄₅NO₆S, CAS: 516-50-7). GDCA ist pharmazeutisch bereits als Choleretikum in Einsatz gekommen. In dieser zuckergebundenen Form ist DCA im Rahmen einer immunologischen Modulation nicht verwendbar. GDCA und TDCA werden jedoch im Körper zu einem bestimmten Prozentsatz wieder in freie Desoxycholsäure überführt, welche dann den erfindungsgemäßen Zweck erfüllt. Dieses Verhalten kann in Formulierungen fokussiert auf GDCA genutzt werden, um stärker retardierende Effekte eines Präparats zu erwirken.

Die Erkrankung an einem Coronavirus kann in Phasen eingeteilt werden, wobei es völlig individuell ist, wie viele Phasen ein Patient durchläuft. Bezogen auf das SARS-CoV-2 Virus werden drei wiederkehrend angetroffene Phasen der COVID-19 Erkrankung beschrieben (42):
1. Phase I 1-6 Tag Frühe Infektion
2. Phase II ab 7-10 Tag Pulmonale Phase (für 20% der Infizierten)
3. Phase III nach Phase II Hyperinflammatorische Phase (für < 5% der Infizierten)

Eine antivirale Therapie wird generell nur für die Phase I und den Beginn der Phase II (frühe pulmonale Phase) bis zum etwa zehnten Tag nach Symptombeginn empfohlen (42).

Der erfindungsgemäße Einsatz von DCA und/oder deren Verbindungen in der Behandlung einer Corona-Erkrankung erfolgt im Zeitfenster von Tag 1-4 ab Symptombeginn, wo Immunprozesse des unspezifischen Immunsystems dominieren. Bei COVID-19 wäre das die Phase I (infektiöse Erkrankungsphase), in der noch Virenvermehrung stattfindet.

Ab dem 4. Tag wird das unspezifische Immunsystem immer mehr durch das spezifische abgelöst, wodurch andere Immunzellen in den Vordergrund rücken. Ab diesem Zeitpunkt verliert der Einsatz von Desoxycholsäure seine Wirksamkeit und seinen Sinn. Dies entspricht bei COVID-19 der möglichen Phase II (pulmonale Phase).

In der möglichen Phase III von COVID-19 sind autoimmune Reaktionen, etwa als Hyperinflammationssyndrom, teils selbst das Problem. Hier ist der Einsatz von Desoxycholsäure kontraindiziert.

Die Behandlungsdauer erfolgt optimalerweise über einen Zeitraum von 3-4 Tagen, wenn das ganze Zeitfenster der Aktivität des unspezifischen Immunsystems ausgenutzt werden kann.

Die orale Gabe (Tabletten, Kapseln, etc.) wird bevorzugt.

Besteht die Möglichkeit, wird die therapeutische Tagesdosis individuell für einen jeden Patienten abgestimmt. Hierbei spielen Faktoren wie Applikationsform, Körpergewicht, natürlicher DCA-Spiegel des Patienten sowie interferierende Medikamente eine Rolle. Ohne Möglichkeit oder Zeit zu einer individuellen Abstimmung, beträgt die Tagesdosis des Wirkstoffs Desoxycholsäure (DCA) oder von Natriumdesoxycholat (NaDCA) bei oraler Einnahme einem Äquivalent von 6 mg/kg Körpergewicht. Die Tagesdosis ist so eingestellt, dass hinreichend die aktuell fehlende, sonst natürlich im Körper produzierte und vorhandene DCA substituiert wird.

Da der Körper einen immanenten Regulationsmechanismus für die Konzentration von Desoxycholsäure, deren Salze und Konjugate hat, sind bei der Verabreichung Abweichungen in einer Größenordnung von 10% von der empfohlenen Tagesdosis in der Anwendung medizinisch vertretbar.

Bei der oralen Applikation wird die Tagesdosis in einer Gabe verabreicht. Die langsame Resorption im Magen-Darm-Trakt hat eine natürliche Retardierungsfunktion, womit ein langsam ansteigender sowie abfallender DCA-Spiegel erreicht wird. Das DCA-haltige Präparat wird zusammen mit einer Mahlzeit eingenommen, damit eine Resorption schonend erfolgen kann.

Empfohlen wird aufgrund des Verdauungszyklus eine Mahlzeit zur Mitte des Tages (Mittagessen), somit im Dosierungsschema für den Patienten Morgens-Mittags-Abends von 0-1-0. Eine Dosierung zu einer anderen Tageszeit (1-0-0 oder 0-0-1) ist jedoch auch ausreichend wirksam.

Bekannt sind Wechselwirkungen zwischen Desoxycholsäure und Kortikosteroiden, da beide eine verwandte Molekülstruktur haben und hier eine Konkurrenz an gleichen Rezeptoren (auch der Immunzellen) besteht.

DCA und Kortikosteroide sind daher antagonistisch aktiv. Während DCA Entzündungen und Aktivität des unspezifischen Immunsystems stimuliert, senken Kortikosteroide beides. Kortikosteroide blockieren antagonistisch die immunologische Wirkung von DCA und umgekehrt. Kortikoide sind deshalb bei Viruserkrankungen wie Erkrankungen mit dem Herpes-Virus bekanntermaßen kontraindiziert. Auch bei Erkrankungen mit dem CoV-Virus wurde dieser Zusammenhang befunden - Kortikosteroide verlangsamen dort den Abbau der Virenlast (37) (35). Eine vermeidbare Dosierung von Kortikosteroiden wäre daher während einer Behandlung mit Desoxycholsäure auszusetzen.

Eine antagonistische Sachlage gilt der aktuellsten Forschung nach auch für die Wechselwirkung mit Urso-Desoxycholsäure (UDCA) (43). Dies ist daher für die Behandlung einer Corona-Erkrankung mit DCA-haltigen Präparaten in Phase I kontraindiziert. Da UDCA pharmazeutisch jedoch selten verwendet wird, wird man in der Praxis auf diese Wechselwirkung kaum stoßen.

Neben den Menschen besitzen ebenso bestimmte Tierarten ein Immunsystem, welches Desoxycholsäure als Immunmodulator nutzt. Ein auf Desoxycholsäure reagierendes unspezifisches Immunsystem wird vorgefunden bei einer Reihe von "höheren Säugetieren", darunter etwa Pferden, Rindern, Schweinen, Hunden oder auch Meerschweinchen.

Bei diesen Tieren kann eine Behandlung entsprechend der grundlegenden Leitlinien (Behandlungsbeginn, Behandlungsdauer) für den Menschen auf das spezifische Tier adaptiert werden. Variiert wird hier Applikationsform sowie eine dem Metabolismus des jeweiligen Tieres gerechte Tagesdosis und deren Verteilung.

### Beschreibung der Ausführungsarten

### Anwendungsbeispiel 1

Behandelt wird ein 60-jähriger Mann mit einem Körpergewicht von 80 kg. Der Patient ist in einer Arztpraxis vorstellig und benennt unklare Erkältungssymptome, welche am Vortag entstanden sind und über Nacht schlimmer wurden. Ein PCR-Schnelltest für CoV (hier SARS-CoV-2) ermittelt innerhalb einer Stunde ein positives Ergebnis, womit eine Corona-Erkrankung mit einer hinreichenden Wahrscheinlichkeit vorliegt. Da sich der Patient am Beginn der Phase I befindet, ist eine erfindungsgemäße Behandlung zur Hebung der unspezifischen Immunabwehr klar indiziert.

Es wird frühestmöglich beginnend eine Behandlung mit einem Präparat mit dem erfindungsgemäßen Wirkstoff (hier DCA) angesetzt. Hierbei werden Formulierungen in Gelatinekapseln mit einer Wirkstoffmenge von 100 mg DCA verwendet. Die Tagesdosis für diesen Patienten beträgt 480mg (80 x 6 mg). Diese wird ihm in der Form von fünf Kapseln Mittags (Schema 0-1-0) im Rahmen einer Mahlzeit verordnet. Somit wird ihm eine Tagesdosis von 500 mg zugeführt, welche sich im Toleranzbereich von 10% von der empfohlenen Dosis bewegt.

Die Behandlung wird über einen Zeitraum von drei Tagen durchgeführt und dann beendet. Damit wird der Tag 2-4 in der ersten Phase einer Corona-Erkrankung ausgenutzt, wo das unspezifische Immunsystem stark aktiv ist.

### Anwendungsbeispiel 2

Behandelt wird eine 35-jährige Frau mit einem Körpergewicht von 60 kg. Die Patientin ist in einer Arztpraxis vorstellig und benennt unklare Erkältungssymptome, welche am Vortag entstanden sind und über Nacht schlimmer wurden. Zur Abklärung wird ein gängiger PCR-Test für CoV (hier SARS-CoV-2) vorgenommen, dessen positives Ergebnis 48 Stunden später vorliegt. Aktuell verwendet die Patientin bedarfsweise ein Cortison-Nasenspray, weil eine Anfälligkeit für Heuschnupfen besteht. Da sich die Patientin noch in der Phase I befindet, ist eine erfindungsgemäße Behandlung (hier DCA) zur Hebung der unspezifischen Immunabwehr noch indiziert.

In ärztlicher Absprache mit ihr wird Cortison während der Behandlungsdauer abgesetzt, da dies für alle Beteiligten medizinisch vertretbar ist und die Vorteile die möglichen Nachteile überwiegen. Hierbei werden Formulierungen in Gelatinekapseln mit einer Wirkstoffmenge von 50 mg DCA verwendet. Die Tagesdosis für diese Patientin beträgt 360mg (60 x 6 mg). Hierbei werden von ihr 7 Kapseln zu einer Mahlzeit eingenommen. Somit wird ihr eine Tagesdosis von 350 mg zugeführt, welche sich im Toleranzbereich von 10% von der empfohlenen Dosis bewegt. Es wird der frühestmögliche Behandlungsbeginn gewählt, da das Behandlungs-Zeitfenster relativ kurz ist. Der Dosierungsmodus kann daher 1-0-0, 0-1-0 oder 0-0-1 sein, je nachdem wann am Tage ärztlicherseits die Behandlungsempfehlung erfolgt ist.

Die Behandlung wird über einen Zeitraum von 1-2 Tagen durchgeführt und dann beendet. Damit wird der Tag 4-5 in der ersten Phase einer Corona-Erkrankung ausgenutzt. Am Tag vier nimmt das unspezifische Immunsystem in dessen Aktivität deutlich ab, während das spezifische Immunsystem übernimmt. Am fünften Tag ist das unspezifische Immunsystem durch Desoxycholsäure nur noch wenig stimulierbar.

### Anwendungsbeispiel 3

Behandelt werden soll ein 70-jähriger Mann mit einem Körpergewicht von 70 kg. Der Patient befindet sich aufgrund einer diagnostizierten CoV-Erkrankung (hier SARS-CoV-2) auf der Intensivstation eines Krankenhauses. Der Erkrankungsbeginn liegt länger als zehn Tage zurück und der Patient befindet sich in der pulmonalen Phase, Beatmungshilfen sind erforderlich. Aufgrund der schlechten Konstitution des Patienten (Multimorbidität, gehobenes Alter) besteht zudem die Gefahr eines Abrutschens in die hyperinflammatorische Phase.

Eine Behandlung mit einem Präparat welches Desoxycholsäure, deren Salze oder Konjugate enthält, ist kontraindiziert. Zum einen besteht hier praktisch keine Möglichkeit zur Stimulierung der unspezifischen Immunabwehr, welche in dieser Phase einer CoV-Erkrankung in den Hintergrund getreten ist. Zum anderen besteht hier die Gefahr einer kontraproduktiven Promotion von autoimmunen Effekten, was die Wahrscheinlichkeit des Abrutschens des Patienten in eine hyperinflammatorische Phase erhöhen könnte.

### Gewerbliche Anwendbarkeit

Technisch wird Desoxycholsäure heutzutage durch alkalische Hydrolyse aus Rindergalle (teils auch Schweinegalle) hergestellt und der pharmazeutischen Industrie zur Verfügung gestellt. Eine synthetische Herstellung wurde bisher aus Kostengründen) nicht verfolgt, wird jedoch künftig angestrebt (Pat. US 2013190283A1, 2013). Die weltweite Verfügbarkeit von Desoxycholsäure ist daher in breitem Maße gewährleistet, da die Gewinnung von Gallensäuren als Beiprodukt bei der Schlachtung in entsprechendem Maße erfolgt.

Es gibt mehrere praktikable Zubereitungs- und Applikationsformen, mit welchen der Wirkstoff dem Patienten verabreicht werden kann. Unser Darmtrakt ist mit DCA und deren Verbindungen als dort natürlich vorkommenden Substanzen vertraut, womit orale Applikationsformen körpergerecht sind und favorisiert werden. Eine Herstellung erfindungsgemäßer Formulierungen aus den Rohstoffen ist im Falle der Verwendung der soliden Form der Desoxycholsäure oder zielführender Verbindungen zur oralen Gabe von geringer Komplexität und könnte schnell umgesetzt werden.

Praktikabel ist die Abfüllung vom Wirkstoff Desoxycholsäure (DCA) oder einem Äquivalent an Natriumdesoxycholat (NaDCA) in Gelatinekapseln. Eine Pressung in Tabletten ist ebenso technisch möglich. Technisch sinnvoll ist die Herstellung von Tabletten oder Kapseln mit 50 mg sowie 100 mg Wirkstoff, damit eine praktikable sowie hinreichend feine Dosierung erfolgen kann.

Eine parenterale Verabreichungsform, etwa wie intravenös, ist hingegen weder notwendig noch angezeigt. Diese würde keine Vorteile bringen. Von einer subkutanen Verabreichung wird zudem abgeraten. Diese hat die Gefahr einer unnötigen lokalen Reizung des umliegenden Gewebes, welches auf diese dort plötzlich massiv überhöhte DCA-Konzentration, die sich nicht schnell verteilen kann, nicht eingestellt ist.

Feststoffbasierte Formulierungen zeigen zudem bei sachgemäßer Lagerung und Anwendung eine gute Resistenz gegenüber Umwelteinflüssen und eine für viele Einsatzzwecke geeignete Haltbarkeit. Somit können DCA-basierte Präparate sowie passende Formulierungen auch in Regionen der Welt eingesetzt werden, wo Bedingungen für Transport und Lagerung nicht etwa dem europäischen Standard entsprechen.

### Liste der angegebenen Unterlagen

1. Ksiazek, T. und et. al. A novel coronavirus associated with severe acute respiratory syndrome. N. Engl. J. Med. 348. 2003, S. 1953-1966.
2. Zaki, A.-M. et al. Isolation of a novel coronavirus from a man with pneumonia in Saudi Arabia. N. Engl. J. Med. 367. 2012, S. 1814-1820.
3. Du Troit, A. Outbreak of a novel coronavirus. Not. Rev. Microbiol. 2020.
4. Wu, A. et al. Genome Composition and Divergence of the Novel Coronavirus (2019-nCoV) Originating in China. Cell Host Microbe. 2020.
5. Zhou, P. et al. A pneumonia outbreak associated with a new coronavirus of probable bat origin. Nature 2020 579 (270-273). [Online] 03. 02 2020. https://www.nature.com/articles/s41586-020-2012-7.
6. FLI. Tierseuchengeschehen und Corona. Friedrlich Löffler Institut. [Online] 24. 09 2020. https://www.fli.de/de/aktuelles/tierseuchengeschehen/coronavirus/.
7. RKI. SARS-CoV-2 Steckbrief zur Coronavirus-Krankheit-2019 (COVID-19). Robert Koch Institut. [Online] 18. 09 2020. https://www.rki.de/DE/Content/InfAZ/N/Neuartiges_Coronavirus/Steckbrief.html#doc13776792bo dyText1.
8. Huang, C. Clinical features of patients infected with 2019 novel coronavirus in Wuhan, China. The Lancet 395 (10223). 2020, S. 497-506.
9. WHO. WHO Director - General's remarks at the media. WHO (World Health Organisation) International. [Online] 11. 02 2020. https://www.who.int/dg/speeches/detail/who-director-general-s-remarks-at-the-media-briefing-on-2019-ncov-on-11-february-2020.
10. Wu, Z. et al. Characteristics of and Important Lessons from the Coronavirus Disease 2019 (COVID-19) Outbreak in China. JAMA - Journal of the American Medical Association. 2020.
11. JHU. COVID-19 Dashboard. CSSE at Johns Hopkins Univ. [Online] 20. 09 2021. https://gisanddata.maps.arcgis.com/apps/dashboards/bda7594740fd40299423467b48e9ecf6.
12. PubChem. Deoxycholic Acid (compound summary). NIH National Library Mdeicine US - PubChem. [Online] 20. 09 2020. https://pubchem.ncbi.nlm.nih.gov/compound/Deoxycholicacid#section=Use-and-Manufacturing.
13. WHO. Deoxycholic Acid. JECFA (World Health Organisation). [Online] 15. 09 2020. https://apps.who.int/food-additives-contaminants-jecfa-database/chemical.aspx?chemID=2948.
14. Allergan Inc. KYBELLA® (deoxycholic acid) injection, for subcutaneous use. Allergan Inc. [Online] 15. 09 2020. https://www.allergan.com/products/kybella.
15. Chen, X. et al. Regulatory effects of deoxycholic acid, a component of the anti-inflammatory traditional Chinese medicine Niuhuang, on human leukocyte response to chemoattractants. Biochemical Pharmacology, Issue: 3, Volume: 63, Pages: 533-541. [Online] 01. 02 2002. https://www.lens.org/lens/scholar/article/054-081-098-664-303/main.
16. Wang, H., et al. Microbial metabolite deoxycholic acid controls Clostridium perfringens-induced chicken necrotic enteritis through attenuating inflammatory cyclooxygenase signaling. Nature - Scientific Reports Art. 14541. [Online] 10. 10 2019. https://www.nature.com/articles/s41598-019-51104-0.
17. Chung, S.-J. et al. The role of phosphatidylcholine and deoxycholic acid in inflammation. NIH - PubMed - Life Science 108(2) 88-93. [Online] 29. 05 2014. https://pubmed.ncbi.nlm.nih.gov/24880073/.
18. Vlcek, B. Potentiace imunitní odpovědi kyselinou desoxycholovou. Prkatický Lekav̌ řě. 1972, S. 326-330.
19. Vlcek, B. Perspektivy steroidních immunopromotorú. Ceský Fyziol. 23. 1974, S. 371-372.
20. Chýle, M. Affecting the immunity response with deoxycholic acid. NIH - PubMed. [Online] 07 1982. https://pubmed.ncbi.nlm.nih.gov/7134784/.
21. Vavassori, P. et al. The bile acid receptor FXR is a modulator of intestinal innate immunity. The Journal of Immunology 183 (10) 6251-6261. [Online] 11 2015. https://www.jimmunol.org/content/183/10/6251.
22. Fiorucci, S. et al. Counter-Regulatory Role of Bile Acid Activated Receptors in Immunity and Inflammation. Bentham Science - Current Molecular Medicine Vol. 10, Issue 6. [Online] 2010. https://www.eurekaselect.com/72226/article.
23. Chen, M.-L. et al. Emerging roles of bile acids in mucosal immunity and inflammation. Mucosal Immunology 12, 851-861. [Online] 05. 04 2019. https://www.nature.com/articles/s41385-019-0162-4.
24. Fiorucci, S. et al. Bile Acids Activated Receptors Regulate Innate Immunity. Frontiers in Immunology Vol. 9, Art. 1853. [Online] 13. 08 2018. https://www.frontiersin.org/articles/10.3389/fimmu.2018.01853/full.
25. Chyle, M. et al. Deoxycholic Acid in the therapy of herpes labialis. Casopis Cesky Lekar 114-40. 1975, S. 1225-1229.
26. Bradna, J. Zur Behandlung des Herpes Zoster mit Desoxycholsäure und Rehabilitation. Rehabilitacia 16-2 (SLO). 1983, S. 77-88.
27. Mroz, M.-S., et al. The bile acids, deoxycholic acid and ursodeoxycholic acid, regulate colonicepithelial wound healing. American Journal of Physiology-Gastrointestinal and Liver Physiology, Vol. 314, No. 3. [Online] 08. 03 2018. https://journals.physiology.org/doi/full/10.1152/ajpgi. 00435.2016.
28. Lacaille, F. et al. The immunosuppressive effect of ursodeoxycholic acid: a comparative in vitro study on human peripheral blood mononuclear cells. Hepatology 18, 165-172. [Online] 07 1993. https://aasldpubs.onlinelibrary.wiley.com/doi/abs/10.1002/hep.1840180125?sid=nlm%3Apubmed
29. Fobar, R. China promotes bear bile as coronavirus treatment, alarming wildlife advocates. National Geographie. [Online] 25. 03 2020. https://www.nationalgeographic.com/animals/2020/03/chinese-government-promotes-bear-bileas-coronavirus-covid19-treatment/.
30. Hagrey, Lee R. und al., et. Ursodeoxycholic acid in the Ursidae. [Hrsg.] Department of Medicine, Univ. of California Division of Gastroenterology. Journal of Lipid Research. San Diego : s.n., 1993, Bd. 34, S. 1911-1917.
31. Subramanian, S. und al., et. Merit of an Ursodeoxycholic Acid Clinical Trial in COVID-19 Patients. Vaccines (Basel) 8(2) 320. [Online] 19. 07 2020. https://www.mdpi.com/2076-393X/8/2/320.
32. Abdulrab, S. et al. Ursodeoxycholic acid as a candidate therapeutic to alleviate and prevent COVID-19-associated cytokine storm. Science Direct - Med Hypotheses. [Online] 30. 05 2020. https://www.sciencedirect.com/science/article/pii/S0306987720312275?via%3Dihub.
33. Zumla, A. et al. Coronaviruses - drug discovery and therapeutic options. Nat. Rev. Drug Discovery 15. 2016, S. 327-347.
34. Feldt, T. et al. Gibt es antivirale Substanzen, die zur Behandlung von COVID-19 zur Verfügung stehen? (DOI 10.25646/6953.2). STAKOB (RKI). [Online] 08. 07 2020. https://www.rki.de/DE/Content/InfAZ/N/Neuartiges_Coronavirus/COVRIIN.html.
35. Feldt, T. et al. Diagnostik und Therapie von Patienten mit COVID-19 (DOI 10.25646/6539.14). STAKOB (RKI). [Online] 06. 08 2020. https://www.rki.de/DE/Content/InfAZ/N/Neuartiges_Coronavirus/Therapie/Therapie_Tab.html.
36. Feldt, T. et al. Wie ist die aktuelle Datenlage zur Behandlung von COVID-19 mit Remdesivir? (DOI 10.25646/6939.5). STAKOB (RKI). [Online] 25. 08 2020. https://www.rki.de/DE/Content/InfAZ/N/Neuartiges_Coronavirus/COVRIIN.html.
37. Yang, Y. et al. Traditional Chinese Medicine in the Treatment of Patients Infected with 2019-New Coronavirus (SARS-CoV-2): A Review and Perspective. Int. Jour. of Biol. Sciences 16 (10). 2020, S. 1708-1717.
38. Fischer, L. Schwere Covid-19-Verläufe Wie der eigene Körper den Weg für das Coronavirus frei macht. ZEIT ONLINE. [Online] 28. 09 2020. https://www.zeit.de/wissen/gesundheit/2020-09/schwere-covid-19-verlaeufe-studie-immunschwaeche-genetisch-bedingt.
39. Paul Ehrlich Institut. COVID-19-Impfstoffe. Paul Ehrlich Institut. [Online] [Zitat vom: 22. 09 2021.] https://www.pei.de/DE/arzneimittel/impfstoffe/covid-19/covid-19-node.html.
40. WHO. COVID-19 vaccine tracker and landscape. World Health Organisation. [Online] [Zitat vom: 22. 09 2021.] https://www.who.int/publications/m/item/draft-landscape-of-covid-19-candidate-vaccines.
41. Israel, A. und al., et. Large-scale study of antibody titer decay following BNT162b2 mRNA vaccine or SARS-CoV-2 infection. Tel Aviv, Israel : Leumit Research Institute, 2021.
42. Feldt, T. et al. Wann könnte die Einleitung einer antiviralen Therapie gerechtfertigt werden? (DOI 10.25646/6962.2). STAKOB (RKI). [Online] 27. 07 2020. https://www.rki.de/DE/Content/InfAZ/N/Neuartiges_Coronavirus/COVRIIN.html.
43. Abdel-Latif, M. et al. Opposing effects of bile acids deoxycholic acid and ursodeoxycholic acid on signal transduction pathw. European Journal of Cancer Prevention: September 2016 - Volume 25 - Issue 5 - p 368-379. [Online] 2016. https://journals.lww.com/eurjcancerprev/Abstract/2016/09000/Opposing_effects_of_bile_acids_d eoxycholic_acid.2.aspx.

## Patentansprüche

1. Desoxycholsäure und/oder deren Verbindungen zur Verwendung bei der Behandlung von Erkrankungen, **dadurch gekennzeichnet, dass** gleichzeitig
a. die Erkrankung durch das Corona-Virus (CoV) verursacht wird,
b. Desoxycholsäure und/oder deren Verbindungen in pharmazeutisch wirksamer Menge verwendet werden,
c. zur Verwendung Desoxycholsäure oder deren folgende Verbindungen kommen: Salze der Desoxycholsäure (Desoxycholate), zuckergebundene Konjugate der Desoxycholsäure.

2. Verwendung nach Anspruch 1 in Formulierungen, welche mindestens einen der folgenden drei Bestandteile in pharmazeutisch wirksamer Menge enthalten: Desoxycholsäure, Salze der Desoxycholsäure (Desoxycholate), zuckergebundene Konjugate der Desoxycholsäure.

3. Verwendung nach Anspruch 1 und 2 **dadurch gekennzeichnet, dass** die pharmazeutischen Formulierungen im Humanbereich und im Veterinärbereich eingesetzt werden.

4. Verwendung nach Anspruch 1 und 2 **dadurch gekennzeichnet, dass** eine Formulierung ein Arzneimittel, ein Nahrungsergänzungsmittel oder ein mit Gallensäuren gemäß Anspruch 1 angereichertes Nahrungsmittel / Futtermittel / Kosmetikum ist.
